# EUROPEAN PATENT APPLICATION

(11) **EP 1 550 665 A1**
(43) Date of publication of application: **06.07.2005**
(21) Application number: 04030327.3
(22) Date of filing: 21.12.2004
(51) Int. Cl.: C07H 1/00, C07H 19/16

(54) **A process for the production of purine nucleoside compounds**

(30) Priority: 26.12.2003 JP 2003434009
(71) Applicant: Ajinomoto Co., Inc., Tokyo 104-8315 (JP)
(72) Inventor: Torii, Takayoshi, Kawasaki-shi Kanagawa (JP); Onishi, Tomoyuki, Kawasaki-shi Kanagawa (JP); Izawa, Kunisuke, Kawasaki-shi Kanagawa (JP)
(74) Representative: Strehl Schübel-Hopf & Partner

(57) **Abstract**

The invention provides a process wherein a 3'-deoxy-3'-halopurine nucleoside compound is treated with perfluoroalkanesulfonyl fluoride in the presence of a base to give a 2',3'-didehydro-2',3'-dideoxypurine nucleoside compound. The resulting compound may be subjected to a catalytic hydrogenolysis to obtain a 2',3'-dideoxypurine nucleoside compound.

## Description

### Technical Field

The present invention relates to a process for the production of a 2',3'-didehydro-2',3'-dideoxypurine nucleoside compound and a 2',3'-dideoxoypurine nucleoside compound and it also relates to intermediate compounds which are useful for the production of those compounds.

### Background Art

A 2',3'-didehydro-2',3'-dideoxypurine nucleoside compound (hereinafter, may be abbreviated as a D4 compound) represented by cyclo D4G (see WO 02/062,123) and 2',3'-dideoxypurine nucleoside compound (hereinafter, may be abbreviated as a DD compound) represented by DDI are compounds useful as pharmaceuticals such as anti-HIV drug.

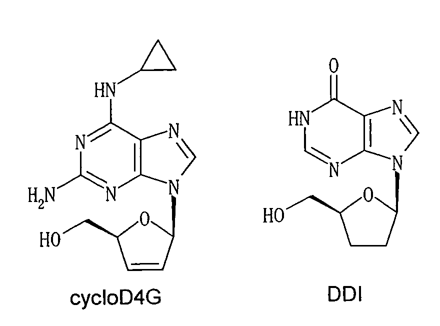

For example, with regard to a process for the production of a D4 compound, a process where an acetoxybromo compound of a purine nucleoside is reduced with zinc is reported in a *Tetrahedron Letters*, (England), 1984, Volume 25, pages 367-370. However, in this method, it is necessary to add zinc metal of more than a stoichiometric amount. It is difficult to remove zinc from the reaction solution after completion of the reaction and, in addition, a large amount of waste zinc is by-produced whereby that is not a preferred method in view of environment. Further, in *Journal of Organic Chemistry*, (U. S. A.), 1989, Volume 54, pages 2217-2225, a method where a 2',3'-dithiocarbonyl compound of purine nucleoside is subjected to a radical reduction is reported. However, since a radical reduction is carried out in this reaction, it is necessary that protection and deprotection of a hydroxyl group are repeated and there is a problem that yield of the desired product significantly lowers.

### Disclosure of the Invention

### Problems that the Invention is to Solve

An object of the present invention is to provide a process by which a 2',3'-didehydro-2',3'-dideoxypurine nucleoside compound and a 2',3'-dideoxypurine nucleoside compound are efficiently produced.

### Means for Solving the Problems

The present inventors have found to their surprise that, when a 3'-deoxy-3'-halopurine nucleoside compound is treated with perfluoroalkanesulfonyl fluoride in the presence of a base, the corresponding 2',3'-didehydro-2',3'-dideoxypurine nucleoside is able to be prepared in a single step in a high yield whereupon the present invention has been achieved. Said 2',3'-didehydro-2',3'-dideoxypurine nucleoside compound is able to be easily made into the corresponding 2',3'-dideoxypurine nucleoside when the double bond is subjected to a catalytic hydrogenolysis.

The present invention provides the following:
(1) A process for the production of a 2',3'-didehydro-2',3'-dideoxypurine nucleoside compound represented by formula (2) 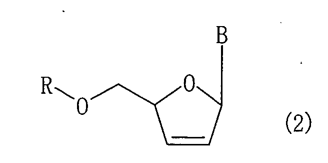 characterized in that a 3'-deoxy-3'-bromopurine nucleoside compound represented by formula (1) 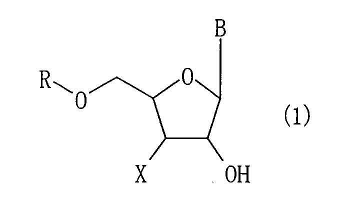 is treated with perfluoroalkanesulfonyl fluoride in the presence of a base,
   wherein in the above formulae, X is a chlorine, bromine or iodine atom, R is a protective group for hydroxyl group, and B is a purine base.
(2) A process for the production of a 2',3'-dideoxypurine nucleoside compound represented by formula (3) 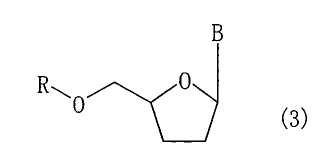 characterized in that a 2',3'-didehydro-2',3'-dideoxypurine nucleoside compound represented by the formula (2) is produced according to a production process mentioned in (1) and then it is subjected to a catalytic hydrogenolysis.
(3) The process for the production according to (1) or (2), wherein the base is a tertiary amine.
(4) A process for the production of a 2',3'-didehydro-2',3'-dideoxypurine nucleoside compound represented by formula (2') 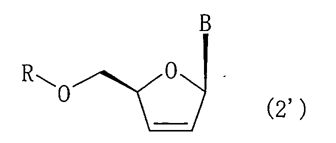 characterized in that a 3'-deoxy-3'β-halopurine nucleoside compound represented by formula (1') 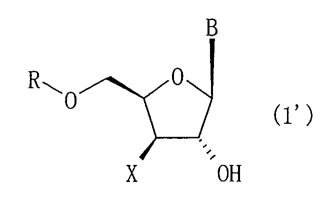 is treated with perfluoroalkanesulfonyl fluoride in the presence of a base,
   wherein in the above formulae, X is a chlorine, bromine or iodine atom, R is a protective group for hydroxyl group, and B is a purine base.
(5) A process for the production of a 2',3'-dideoxypurine nucleoside compound represented by the formula (3') 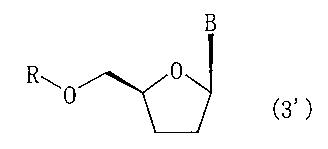 characterized in that a 2',3'-didehydro-2',3'-dideoxypurine nucleoside compound is produced according to a production process mentioned in (4) and then it is subjected to a catalytic hydrogenolysis.
(6) The process for the production according to (4) or (5), wherein the base is a tertiary amine.
(7) N²,5'-O-Diacetyl-3'-deoxy-3'β-bromoguanosine represented by formula (1a) 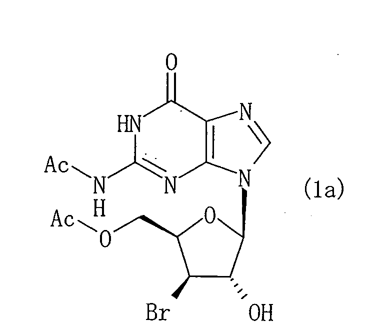 wherein Ac is an acetyl group.
(8) N²,5'-O-Diacetyl-2',3'-didehydro-2',3'-dideoxyguanosine represented by formula (2a) 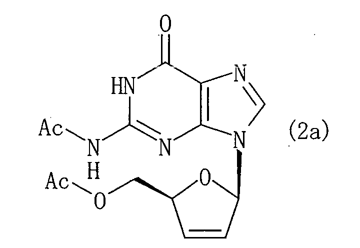 wherein Ac is an acetyl group.
(9) N²,5'-O-Diacetyl-3'-deoxy-3'β-bromo-2-amino-6-chloropurine riboside represented by formula (1b). 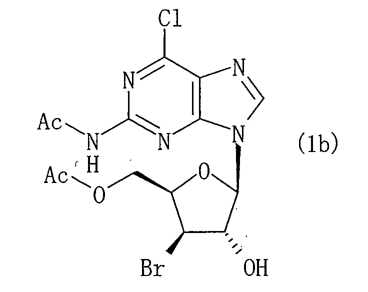 wherein Ac is an acetyl group.
(10) N²,5'-O-Diacetyl-2',3'-didehydro-2',3'-dideoxy-2-amino-6-chloropurine riboside represented by formula (2b). 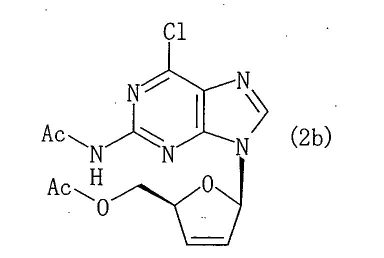 wherein Ac is an acetyl group.
(11) A process of the production of a 2',3'-didehydro-2',3'-dideoxypurine nucleoside compound represented by formula (6) 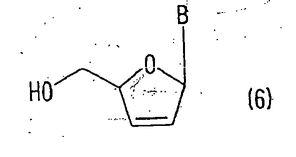 wherein:
   B is a purine base,
      werein said process comprises:
      (a) treating a 3'-deoxy-3'-halopurine nucleoside compound represented by formula (1) 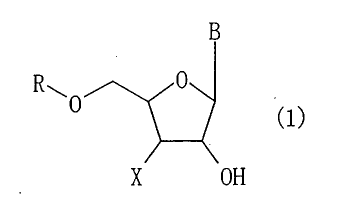 wherein:
         X is a chlorine atom, a bromine atom, or a iodine atom;
         R is a protecting group; and
         B is as defined above;
         with at least one perfluoroalkanesulfonyl fluoride in the presence of at least one base to obtain a 2',3'-didehydro-2',3'-dideoxypurine nucleoside compound represented by formula (2) 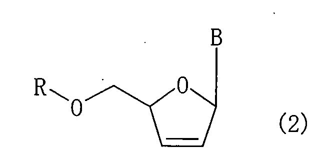 wherein:
         R and B are as defined above; and
      (b) subjecting said 2',3'-didehydro-2',3'-dideoxypurine nucleoside compound of formula (2) to de-protection of R and, if necessary, to at least one of protection, de-protection and modification for a group at the 2-position and/or the 6-position of the purine base.
(12) A process for the production of a 2',3'-didehydro-2',3'-dideoxypurine nucleoside compound represented by formula (6') 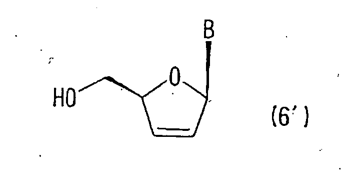 wherein:
   B is a purine base;
   wherein said process comprises:
   (a) treating a 3'-deoxy-3'-halopurine nucleoside compound represented by formula (1') 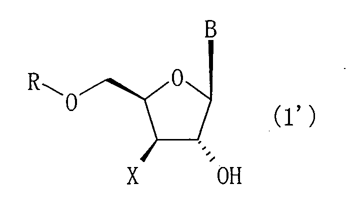 wherein:
      X is a chlorine atom, a bromine atom, or a iodine atom;
      R is a protecting group; and
      B is as defined above;
         with at least one perfluoroalkanesulfonyl fluoride in the presence of at least one base to obtain a 2',3'-didehydro-2',3'-dideoxypurine nucleoside compound represented by formula (2'): 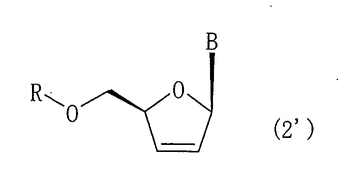 wherein:
      R and B are as defined above; and
   (b) subjecting said 2',3'-didehydro-2',3'-dideoxypurine nucleoside compound of formula (2') to de-protection of R and, if necessary, to at least one of protection, deprotection and modification for a group at the 2-position and/or the 6-position of the purine base.
(13) A process for the production of a 2',3'-dideoxypurine nucleoside compound represented by formula (7) 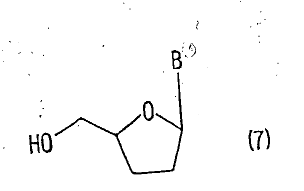 wherein:
   B is a purine base;
   wherein said process comprises:
   (a) treating a 3'-deoxy-3'-halopurine nucleoside compound represented by formula (1): 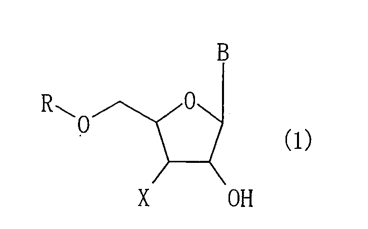 wherein:
      X is a chlorine atom, a bromine atom, or a iodine atom;
      R is a protecting group; and
      B is as deined above;
         with at least one perfluoroalkanesulfonyl fluoride in the presence of at least one base to obtain a 2',3'-didehydro-2',3'-dideoxypurine nucleoside compound represented by formula (2) 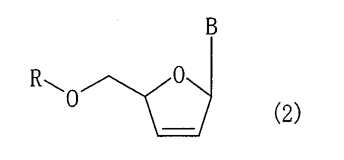 wherein:
      R and B are as defined above;
   (b) subjecting said 2',3'-didehydro-2',3'-dideoxypurine nucleoside compound of formula (2) to catalytic hydrogenation to obtain said 2',3'-dideoxypurine nucleoside compound of formula (3) 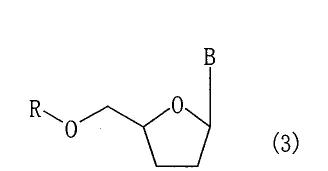 wherein:
      R and B are as defined above; and
   (c) subjecting said 2',3'-dideoxypurine nucleoside compound of formula (3) to de-protection of R and, if necessary, to at least one of protection, de-protection and modification for a group at the 2-position and/or the 6-position of the purine base.
(14) A process for the production of a 2',3'-dideoxypurine nucleoside compound represented by formula (7') 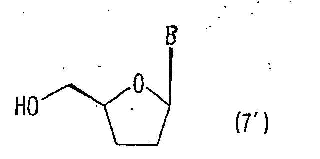 wherein:
   B is a purine base;
   wherein said process comprises:
   (a) treating a 3'-deoxy-3'-halopurine nucleoside compound represented by formula (1'): 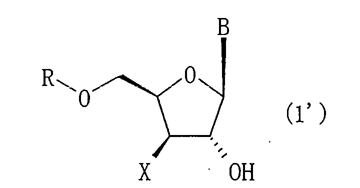 wherein:
      X is a chlorine atom, a bromine atom or a iodine atom;
      R is a protecting group; and
      B is as defined above
         with at least one perfluoroalkanesulfonyl fluoride in the presence of at least one base to obtain a 2',3'-didehydro-2',3'-dideoxypurine nucleoside compound represented by formula (2'): 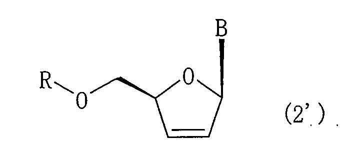 wherein:
      R and B are as defined above;
   (b) subjecting said 2',3'-didehydro-2',3'-dideoxypurine nucleoside compound of formula (2) to catalytic hydrogenation to obtain said 2',3'-dideoxypurine nucleoside compound of formula (3'): 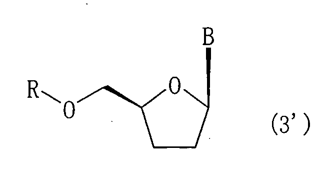 wherein
      R and B are as defined above; and
   (c) subjecting said 2',3'-dideoxypurine nucleoside compound of formula (3) to de-protection of R and, if necessary, to at least one of protection, de-protection and modification for a group at the 2-position and/or the 6-position of the purine base.

### Advantages of the Invention

In accordance with the present invention, it is possible to efficiently produce a 2',3'-didehydro-2',3'-dideoxypurine nucleoside compound and a 2',3'-dideoxypurine nucleoside compound which are useful as pharmaceuticals or intermediates therefor.

### Best Mode for Carrying Out the Invention

In the formulae of the present invention, X is chlorine atom, bromine atom or iodine atom; R is a protective group for hydroxyl group; and B is a purine base.

There is no particular limitation for the protective group for hydroxyl group and its examples are acyl group., alkyl group, aralkyl group and silyl group. Examples of the acyl group are acyl groups having 1 to 7 carbon(s) such as formyl group, acetyl group, pivaloyl group and benzoyl group. Examples of the alkyl group are alkyl groups having 1 to 7 carbons such as methyl group, ethyl group, n-propyl group, isopropyl group, n-butyl group and tert-butyl group. Examples of the aralkyl group are aralkyl groups having 7 to 22 carbons such as benzyl group, trityl group, 4-monomethoxytrityl group and 4,4'-dimethoxytrityl group. Examples of the silyl group are tri-substituted silyl groups such as trimethylsilyl group, triethylsilyl group and tert-butyldimethylsilyl group. Besides those, it is possible to use a protective group for hydroxyl group such as methoxymethyl group, methylthiomethyl group, benzyloxymethyl group, methoxyethoxymethyl group, tetrahydropyranyl group, methoxycarbonyl group, 9-fluorenylmethoxycarbonyl group, 2,2,2-trichloroethoxycarbonyl group, benzyloxycarbonyl group and tert-butoxycarbonyl group. Preferred protective groups for hydroxyl group are acetyl group, benzoyl group, benzyl group and trityl group and acetyl group is particularly preferred.

An example of the purine base is a purine base represented by the following formula (4). 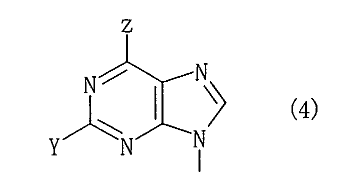 (In the formula, Y and Z may be same or different and each is an optionally substituted amino group, an optionally substituted hydroxyl group, halogen atom or hydrogen atom.)

Examples of the substituent when the amino group is substituted are alkyl group, aralkyl group, acyl group and carbamoyl group. Examples of the alkyl group are alkyl groups having 1 to 10 carbon(s) such as methyl group, ethyl group, n-propyl group, isopropyl group, n-butyl group and tert-butyl group. Examples of the aralkyl group are aralkyl groups having 7 to 22 carbons such as benzyl group, trityl group, 4-monomethoxytrityl group and 4,4'-dimethoxytrityl group. Examples of the acyl group are acyl groups having 1 to 10 carbon(s) such as formyl group, acetyl group, pivaloyl group and benzoyl group. Examples of the carbamoyl group are carbamoyl groups such as benzyloxycarbonyl group, tert-butoxycarbonyl group and methoxycarbonyl group.

Examples of the substituent when the hydroxyl group is substituted are the same ones already mentioned as the protective groups for hydroxyl group.

Examples of the halogen atom are chlorine atom, fluorine atom, bromine atom and iodine atom.

With regard to the purine base, its specific examples are purine, adenine; guanine, N-acetylguanine, hypoxanthine, xanthine, 6-fluoropurine, 6-chloropurine, 6-methylaminopurine, 6-dimethylaminopurine, 6-trifluoromethylaminopurine, 6-cyclopropylaminopurine, 6-benzoylaminopurine, 6-acetylaminopurine, 6-methoxypurine, 6-acetoxypurine, 6-benzoyloxypurine, 6-methylpurine, 6-ethylpurine, 6-trifluoromethylpurine, 6-phenylpurine, 6-mercaptopurine, 6-methylmercaptopurine, 6-aminopurine-1-oxide, 6-hydroxypurine-1-oxide, 2,6-diaminopurine, 2-amino-6-chloropurine, 2-acetylamino-6-chloropurine, 2-aminopurine, 2-amino-6-mercaptopurine, 2-amino-6-methylmercaptopurine, 2-amino-6-hydroxyaminopurine, 2-amino-6-methoxypurine, 2-amino-6-benzoyloxypurine, 2-amino-6-acetoxypurine, 2-amino-6-methylpurine, 2-amino-6-cyclopropylaminopurine and 2-amino-6-phenylpurine.

Preferred purine bases are adenine, guanine, N-acetylguanine, hypoxanthine, 2-amino-6-chloropurine, 2-acetylamino-6-chloropurine and 2-amino-6-cyclopropylaminopurine. Particularly preferred ones are adenine, N-acetylguanine, hypoxanthine and 2-acetylamino-6-chloropurine.

A method for the production of a 2',3'-didehydro-2',3'-dideoxypurine nucleoside compound represented by the formula (2) in the present invention is characterized in that a 3'-deoxy-3'-halopurine nucleoside compound represented by the formula (1) is treated with perfluoroalkanesulfonyl fluoride in the presence of a base.

Examples of the base are alkaline metal hydroxide such as sodium hydroxide, potassium hydroxide and lithium hydroxide; alkaline earth metal hydroxide such as calcium hydroxide; alkaline metal carbonate such as sodium carbonate and potassium carbonate; alkaline earth metal carbonate such as calcium carbonate; alkaline metal hydrogen carbonate such as sodium hydrogen carbonate; and tertiary amine such as trimethylamine, triethylamine, triethylenediamine, N,N-dimethylcyclohexylamine, tetramethylenediamine, N,N,N',N'-tetramethyl-1,3-butanediamine, N-methylmorpholine, N,N-diethyl-2-methylpiperazine and DBU (1,8-diazabicyclo-[5,4,0]undec-7-ene). Tertiary amines are used particularly advantageously.

With regard to the perfluoroalkanesulfonyl fluoride, a perfluoroalkanesulfonyl fluoride represented by the following formula (5) may be exemplified.

P-SO₂-F (5)

(P is a perfluoroalkyl group.)

With regard to the perfluoroalkyl group, a perfluoroalkyl group having 1 to 10 carbon(s) may be preferably exemplified.

Specific examples of the preferred perfluoroalkanesulfonyl fluoride are perfluorobutanesulfonyl fluoride, perfluorohexanesulfonyl fluoride and perfluorooctanesulfonyl fluoride and perfluorobutanesulfonyl fluoride is particularly preferred.

Although there is no particular limitation for the amount of the base used, it is preferably from 1 to 10 or, more preferably, from 2 to 5 in terms of a molar ratio to 1 mol of the 3'-deoxy-3'-halopurine nucleoside compound.

Although there is no particular limitation for the amount of the perfluoroalkanesulfonyl fluoride used, it is preferably from 1 to 10 or, more preferably, from 1 to 5 in terms of a molar ratio to 1 mol of the 3'-deoxy-3'-halopurine nucleoside compound.

Although there is no particular limitation for the ratio of the base to the perfluoroalkanesulfonyl fluoride, it is preferred that, in terms of a molar ratio, the base is from 0.5 to 10 or, more preferably, from 1 to 5, to 1 mol of the perfluoroalkanesulfonyl fluoride.

With regard to a solvent used for the reaction, an aprotic organic solvent may be used and, for example, tetrahydrofuran, dioxane, ethyl acetate, dichloromethane, chloroform, toluene, hexane, acetonitrile, N,N-dimethylformamide, N,N-dimethylacetamide or a mixed solvent of any of them may be used.

Although there is no particular limitation for the concentration of the 3'-deoxy-3'-halopurine nucleoside compound in the reaction solvent in the start of the reaction, it is preferably from 0.05 to 1.0 mol/L or, more preferably, from 0.1 to 0.5 mol/L.

Although there is no particular limitation for the reaction temperature, it may be carried out usually within a range of 20 to 120°C and, preferably, 50 to 80°C. Reaction time is not particularly limited as well and it may be usually carried out for 0.1 to 10 hour(s) and, preferably, 1 to 5 hour(s).

After completion of the reaction, an aimed substance may be isolated and purified by an appropriate use of operations which have been known by persons skilled in the art such as extraction, crystallization and chromatography.

When the resulting 2',3'-didehydro-2',3'-dideoxypurine nucleoside compound is subjected to a catalytic hydrogenolysis, it is able to be made into a 2',3'-dideoxypurine nucleoside compound.

The catalytic hydrogenolysis may be carried out in such a manner that 0.1 to 10 atmosphere(s) (10 to 1,000 kilo-pascal(s)) of hydrogen gas is made to react for 0.1 to 24 hour(s) at the reaction temperature of 0 to 80°C in a solvent such as methanol, acetonitrile, water and N,N-dimethylformamide and in the presence of a catalyst such as palladium carbon, rhodium carbon and ruthenium carbon. The catalyst is filtered off from the resulting reaction solution and the aimed substance is able to be prepared by a method which has been known by persons skilled in the art such as evaporation of the solvent, crystallization and chromatography.

The compound of the formula (1) which is a starting substance may be easily prepared according to the method mentioned in *Nucleosides & Nucleotides*, Volume 15, pages 31 to 45, 1996.

With regard to the 3'-deoxy-3'-halopurine nucleoside compound represented by the formula (1) in the present invention, a 3'-deoxy-3'β-halopurine nucleoside compound represented by the following formula (1') may be advantageously used. 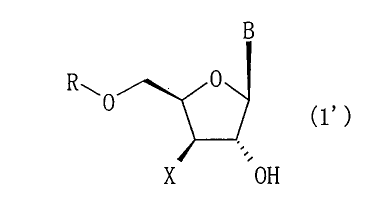 (In the formula, X is chlorine atom, bromine atom or iodine atom; R is a protective group for hydroxyl group; and B is a purine base.)

The symbols X, R and B in the formula are the same as those mentioned already.

As mentioned already, the 3'-deoxy-3'β-halopurine nucleoside compound represented by the formula (1') may be made into a 2',3'-didehydro-2',3'-dideoxypurine nucleoside compound represented by the following formula (2') by the treatment with perfluoroalkanesulfonyl fluoride in the presence of a base according to the present invention. 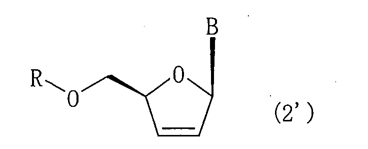 (In the formula, R and B have the same meanings as defined already.)

Further, the 2',3'-didehydro-2',3'-dideoxypurine nucleoside compound represented by the formula (2') is able to be introduced to a 2',3'-dideoxypurine nucleoside compound represented by the formula (3') by a catalytic hydrogenolysis. 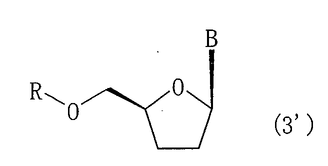 (In the formula, R and B have the same meanings as defined already.)

In the compound of the formula (1'), N²,5'-O-diacetyl-3'-deoxy-3'β-bromoguanosine represented by the following formula (1a) and N²,5'-O-diacetyl-3'-deoxy-3'β-bromo-2-amino-6-chloropurine riboside represented by the following formula (1b) are novel substances.

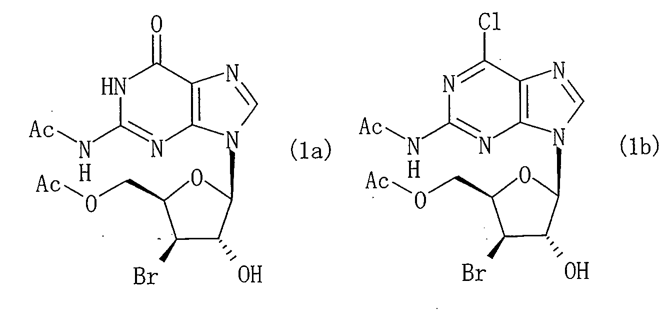

### (In the formula, Ac is acetyl group.)

Further, in the compound of the formula (2'), N²,5'-O-diacetyl-2',3'-didehydroguanosine represented by the following formula (2a) and N²,5'-O-diacetyl-2',3'-didehydro-2',3'-dideoxy-2-amino-6-chloropurine riboside represented by the following formula (2b) are novel substances. 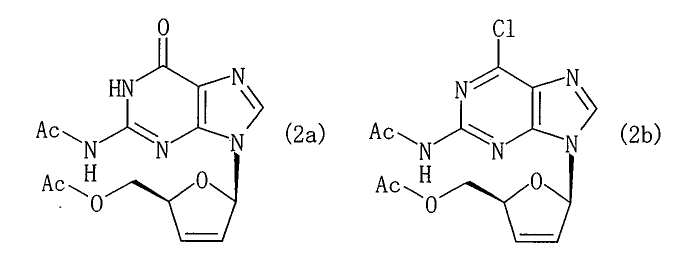 (In the formula, Ac is acetyl group.)

The present invention will now be specifically illustrated by way of the following Examples although the present invention is not limited thereto.

The 2',3'-didehydro-2', 3'-dideoxypurine nucleoside compounds represented by formula (2) and formula (2') may be converted into 2', 3'-didehydro-2', 3'-dideoxypurine nucleoside compounds represented by formula (6) and formula (6') respectively by subjecting compound (2) or (2') to deprotection of R, and if necessary to at least one of protection, de-protection and modification for group at 2-position and/or 6-position of a purine base.

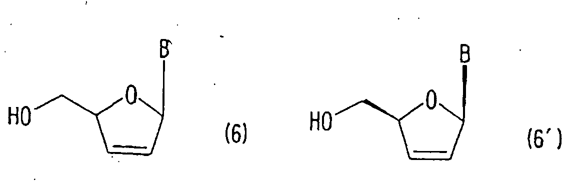

In the formula, B is a purine base. The purine base is the same as described above.

Furthermore, the 2', 3'-dideoxypurine nucleoside compounds represented by formula (3) and formula (3') may be converted into 2', 3'-dideoxypurine nucleoside compounds represented by formula (7) and formula (7') respectively by subjecting compound (3) or (3') to de-protection of R, and if necessary to at least one of protection, de-protection and modification for group at 2-position and/or 6-position of a purine base.

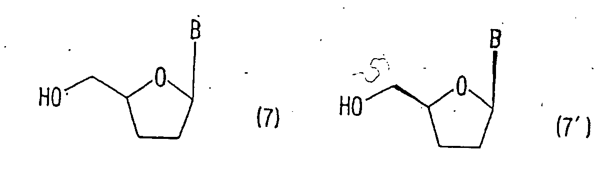

In the formula B is a purine base. The purine base is the same as described above.

The "protecting group" means a group which is intended to be removed after performing requested conversion of another part of compound. The de-protection of protecting group R for hydroxyl group may be performed based on the method described in, for example, Nucleoside & Nucleotides (1998), 7(2), 143-153, Tetrahedron Letters (1988), 29(11), 1239-1243 and the like.

The groups at 2-position and 6-position of purine base correspond to Y and Z in the formula (4) respectively, "If necessary" means,the case that group at 2-position and/or 6-position of purine base is converted to another group.

"Protection" of 2-position and/or 6-position of purine base is, for example, a step of introducing protecting group exemplified above when group at 2-position and/or 6-position of purine base is amino group and/or hydroxyl group. The said "protection" and "de-protection" may be performed by well-known conventional method (for example, see Protective Groups in Organic Synthesis, 3^{rd} edn., Wiley Interscience Publication, John Wiley & Sons, Inc., 1999).

"Modification" shows a conversion of group which is different from above said "protection" and "de-protection". For example, "Modification" means a step of conversion to optional group such as halogen atom and hydrogen atom when group at 2-position and/or 6-position of purine base is amino group and/or hydroxyl group. "Modification" may be performed by well-known conventional method in synthesis of nucleic acid (for example, see, "Nucleic acid Chemistry Part II" Leroy B. Townsend, R. Stuart Tipson, A Wiley Interscience Publication).

### Examples

### <Referential Example 1>

### Production of N²,5'-O-diacetyl-3'-deoxy-3'β-bromoguanosine

To a suspension of N²,2',5'-O-triacetyl-3'-deoxy-3'β-bromoguanosine (944 mg, 2.0 mmol) in ethanol (20 mL) were added hydroxylamine hydrochloride (278 mg, 4.0 mmol) and triethylamine (0.558 mL, 4.0 mmol) followed by stirring for 14 hours at room temperature. Solid was filtered from the resulting suspension and dried *in vacuo* to give an aimed substance (618 mg, yield: 72%) as white solid.
¹H-NMR (DMSO-d6): δ 2.06 (s, 3H), δ 2.19 (s, 3H, δ 4.32-4.40 (m, 2H), δ 4.50-4.57 (m, 1H), δ 4.63 (d-d, 1H, J = 5.2, 3.7 Hz), δ 4.83-4.88 (m, 1H), δ 5.75 (d, 1H, J = 3.8 Hz), δ 6.51-6.55 (m, 1H), δ 8.18 (s, 1H), δ 11.77 (br, 1H), δ 12.10 (br, 1H).
ESIMS m/z: 430 (M + H)

### <Referential Example 2>

### Production of N²,5'-O-diacetyl-3'-deoxy-3'(3-bromo-2-amino-6-chloropurine riboside

To a suspension of N²,2',5'-O-triacetyl-3'-deoxy-3'β-bromo-2-amino-6-chloropurine riboside (260 mg, 0.5 mmol) in ethanol (5 mL) were added hydroxylamine hydrochloride (70 mg, 1.0 mmol) and triethylamine (0.14 mL, 1.0 mmol) followed by stirring for 14 hours at room temperature and the resulting solution was concentrated. An aimed substance (110 mg, yield: 49%) as white solid was prepared by purification by means of chromatography (15 g of silica gel; eluted with hexane-ethyl acetate in 1:2) and drying in *vacuo.*
¹H-NMR (CDCl₃): δ 1.81 (s, 3H), δ 2.29 (s, 3H), δ 4.35-4.41 (m, 1H), δ 4.49 (d-d, 1H, J = 12.4, 3.3 Hz), δ 4.53-4.58 (m, 1H), δ 4.72-4.78 (m, 1H), δ 4.91-4.96 (m, 1H), δ 5.87 (d, 1H, J = 4.3 Hz), δ 6.26 (br, 1H), δ 8.22 (d, 1H, J = 3.6 Hz), δ 8.35 (br, 1H).
ESIMS m/z: 448 (M + M).

### Example 1

### Production of 5'-O-acetyl-2',3'-didehydro-2',3'-dideoxyadenosine

To a solution of 5'-O-acetyl-3'-deoxy-3'β-bromoadenosine (186 mg, 0.5 mmol) in acetonitrile (5 mL) were added triethylamine (0.278 mL, 2.0 mmol) and perfluoro-1-butanesulfonyl fluoride (0.360 mL, 2.0 mmol) followed by stirring for 2 hours with heating at 70°C. The resulting solution was cooled down to room temperature and concentrated *in vacuo.* As a result of purification by chromatography (20 g of silica gel; eluted with dichloromethane-methanol in 32:1), an aimed substance (135 mg, yield: 98%) was prepared as white solid.
¹H-NMR (DMSO-d6): δ 1.99 (s, 3H), δ 4.14-4.21 (m, 2H), δ 5.07-5.12 (m, 1H), δ 6.23-6.28 (m, 1H), δ 6.47-6.52 (m, 1H), δ 6.93-6.97 (m, 1H), δ 7.29 (br, 2H), δ 8.08 (s, 1H), δ 8.17 (s, 1H).
ESIMS m/z: 276 (M + H).

### Example 2

### Production of N²,5'-O-diacetyl-2',3'-didehydro-2',3'-dideoxyguanosine

To a solution of N²,5'-O-diacetyl-3'-deoxy-3'β-bromoguanosine (215 mg, 0.5 mmol) in acetonitrile (5 mL) were added triethylamine (0.278 mL, 2.0 mmol) and perfluoro-1-butane-sulfonyl fluoride (0.360 mL, 2.0 mmol) followed by heating at 70°C for 2 hours with stirring. The resulting solution was cooled down to room temperature and concentrated *in vacuo*. As a result of purification by chromatography (20 g of silica gel; eluted with dichloromethane-methanol in 32:1), an aimed substance (104 mg, yield: 62%) was prepared as white solid.
¹H-NMR (DMSO-d6): δ 1.97 (s, 3H), δ 2.19 (s, 3H), δ 4.14-4.18 (m, 2H), δ 5.07-5.11 (m, 1H), δ 6.25-6.30 (m, 1H), δ 6.50-6.54 (m, 1H), δ 6.76-6.80 (m, 1H), δ 7.90 (s, 1H), δ 11.78 (br, 1H), δ 12.07 (br, 1H).
ESIMS m/z: 334 (M + H).

### Example 3

### Production of 5'-O-acetyl-2',3'-didehydro-2',3'-dideoxyinosine

To a solution of 5'-O-acetyl-3'-deoxy-3'β-bromoinosine (93 mg, 0.25 mmol) in acetonitrile (2.5 mL) were added triethylamine (0.139 mL, 1.0 mmol) and perfluoro-1-butanesulfonyl fluoride (0.180 mL, 1.0 mmol) followed by heating at 70°C for 2 hours with stirring. The resulting solution was cooled down to room temperature and concentrated *in vacuo*. As a result of purification by chromatography (10 g of silica gel; eluted with dichloromethane-methanol in 32:1), an aimed substance (50.6 mg, yield: 73%) was prepared as white solid.
¹H-NMR (DMSO-d6): δ 1.98 (s, 3H), δ 4.16-4.20 (m, 2H), δ 5.08-5.12 (m, 1H), δ 6.24-6.28 (m, 1H), δ 6.50-6.54 (m, 1H), δ 6.91-6.93 (m, 1H), δ 8.02 (s, 1H), δ 8.09 (s, 1H), δ 8.87 (br, 1H).
ESIMS m/z: 277 (M + H).

### Example 4

### Production of N²,5'-O-diacetyl-2',3'-didehydro-2',3'-dideoxy-2-amino-6-chloropurine riboside

To a solution of N²,5'-O-diacetyl-3'-deoxy-3'β-bromo-2-amino-6-chloropurine riboside (63 mg, 0.14 mmol) in acetonitrile (1.0 mL) were added triethylamine (0.057 mL, 0.4 mmol) and perfluoro-1-butanesulfonyl fluoride (0.072 mL, 0.4 mmol) followed by heating at 70°C for 2 hours with stirring. The resulting solution was cooled down to room temperature and concentrated in *vacuo.* As a result of purification by chromatography (20 g of silica gel; eluted with hexane-ethyl acetate in 1:4), an aimed substance (47.5 mg, yield: 97%) was prepared as white solid.
¹H-NMR (CDCl₃): δ 2.08 (s, 3H), δ 2.56 (s, 3H), δ 4.23-4.28 (m, 1H), δ 4.37 (d-d, 1H, J = 12.4, 3.6 Hz), δ 5.16-5.23 (m; 1H), δ 6.15 (d, 1H, J = 4.8 Hz), δ 6.43 (d-d, J = 6.0, 1.6 Hz), δ 7.03-7.09 (m, 1H), δ 8.09 (br, H), δ 8.18 (d, 1H, J = 3.6 Hz).
ESIMS m/z: 374 (M + Na).

### Example 5

### Production of 5'-O-acetyl-2',3'-didehydro-2',3'-dideoxyadenosine

To a solution of 5'-O-acetyl-3'-deoxy-3'β-bromoadenosine (186 mg, 0.5 mmol) in acetonitrile (5 mL) were added triethylamine (0.139 mL, 1.0 mmol) and perfluoro-1-butane-sulfonyl fluoride (0.180 mL, 1.0 mmol) followed by heating at 70°C for 2 hours with stirring. AS a result of quantification by means of an HPLC, it was confirmed that an aimed substance was produced in the resulting solution in an amount of 99 mg (yield: 71%).

### Example 6

### Production of 5'-O-acetyl-2',3'-didehydro-2',3'-dideoxyadenosine

To a solution of 5'-O-acetyl-3'-deoxy-3'β-bromoadenosine (186 mg, 0.5 mmol) in acetonitrile (5 mL) were added triethylamine (0.278 mL, 2.0 mmol) and perfluoro-1-butane-sulfonyl fluoride (0.180 mL, 1.0 mmol) followed by heating at 70°C for 2 hours with stirring. As a result of quantification by means of an HPLC, it was confirmed that an aimed substance was produced in the resulting solution in an amount of 125 mg (yield: 91%).

### Example 7

### Production of 5'-O-acetyl-2',3'-dideoxyadenosine

To a solution of 5'-O-acetyl-2',3'-didehydro-2',3'-dideoxyadenosine (27 mg, 0.1 mmol) in methanol (1 mL) was added 5% palladium carbon (containing 50% of water) (2.7 mg) followed by stirring at room temperature for 14 hours in a hydrogen atmosphere. The palladium catalyst was filtered off from the resulting solution and the filtrate was dried *in vacuo* to give an aimed substance (25 mg, yield: 90%) as white solid.
¹H-NMR (DMSO-d6): δ 1.97 (s, 3H), δ 2.10-2.17 (m, 2H), δ 2.45-2.53 (m, 2H), δ 4.14 (d-d, 1H), J = 11.8, 6.0 Hz), δ 4.22 (d-d, 1H, J = 11.8, 3.1 Hz), δ 4.28-4.30 (m, 1H), δ 6.25 (d-d, 1H, J = 6.8, 3.6 Hz), δ 7.27 (br, 2H), δ 8.15 (s, 1H), δ 8.28 (s, 1H).
ESIMS m/z: 278 (M + H).

A 2',3'-didehydro-2',3'-dideoxypurine nucleoside compound and a 2',3'-dideoxypurine nucleoside compound produced by the production process according to the present invention are able to be advantageously used as pharmaceuticals such as anti-HIV drug or intermediate compounds therefor.

## Claims

1. A process for the production of a 2',3'-d'idehydro-2',3'-dideoxypurine nucleoside compound represented by formula (2) 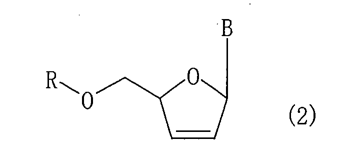 **characterized in that** a 3'-deoxy-3'-halopurine nucleoside compound represented by formula (1) 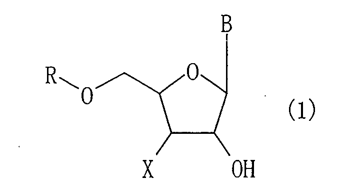 is treated with perfluoroalkanesulfonyl fluoride in the presence of a base,
wherein in the above formulae, X is a chlorine, bromine or iodine atom, R is a protective group for a hydroxyl group, and B is a purine base.

2. A process for the production of a 2',3'-dideoxypurine nucleoside compound represented by formula (3) 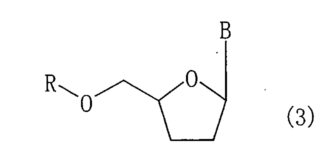 which comprises producing a 2',3'-didehydro-2',3'-dideoxypurine nucleoside compound represented by formula (2) according to the process of claim 1 and then subjecting said compound to a catalytic hydrogenolysis.

3. The process according to claim 1 or 2, wherein the base is a tertiary amine.

4. A process for the production of a 2',3'-didehydro-2',3'-dideoxypurine nucleoside compound represented by formula (2') 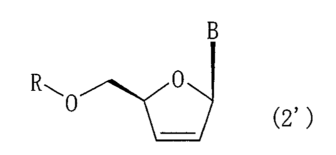 **characterized in that** a 3'-deoxy-3'β'-halopurine nucleoside compound represented by formula (1') 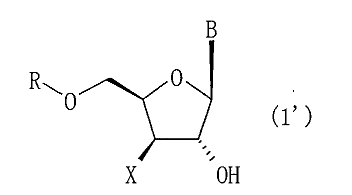 is treated with perfluoroalkanesulfonyl fluoride in the presence of a base,
wherein in the above formulae, X is a chlorine, bromine or iodine atom, R is a protective group for a hydroxyl group, and B is a purine base.

5. A process for the production of a 2',3'-dideoxypurine nucleoside compound represented by formula (3') 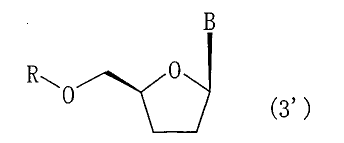 which comprises producing a 2',3'-didehydro-2',3'-dideoxypurine nucleoside compound represented by formula (2') according to the process of claim 4 and then subjecting said compound to a catalytic hydrogenolysis.

6. The process according to claim 4 or 5, wherein the base is a tertiary amine.

7. N²,5'-O-Diacetyl-3'-deoxy-3'β-bromo-guanosine represented by formula (1a) 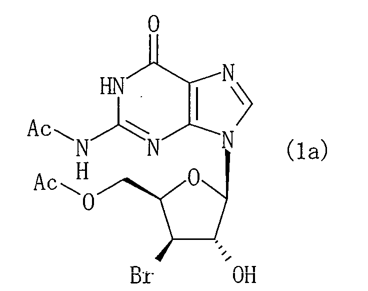 wherein Ac is an acetyl group.

8. N²,5'-O-Diacetyl-2',3'-didehydro-2',3'-dideoxyguanosine represented by formula (2a) 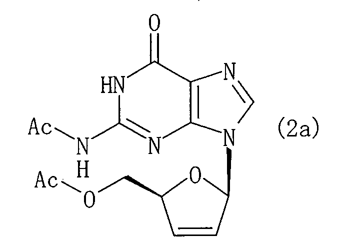 wherein Ac is an acetyl group.

9. N²,5'-O-Diacetyl-3'-deoxy-3'β-bromo-2-amino-6-chloropurine riboside represented by formula (1b) 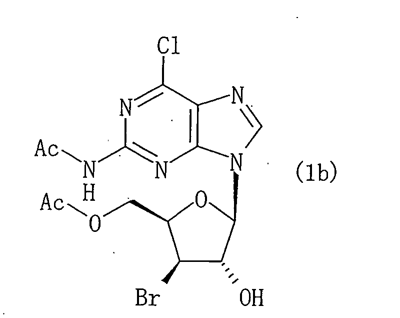 wherein Ac is an acetyl group.

10. N²,5'-O-Diacetyl-2',3'-didehydro-2',3'-dideoxy-2-amino-6-chloropurine riboside represented by formula (2b) 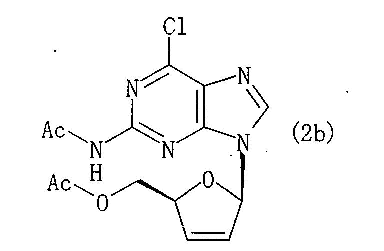 wherein Ac is an acetyl group.

11. A process of the production of a 2',3'-didehydro-2',3'-dideoxypurine nucleoside compound represented by formula (6) 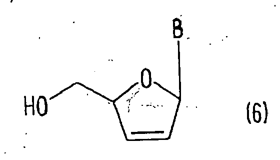 wherein:
B is a purine base,
werein said process comprises:
(a) treating a 3'-deoxy-3'-halopurine nucleoside compound represented by formula (1) 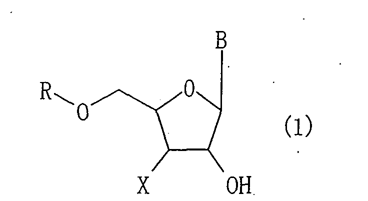 wherein:
X is a chlorine atom, a bromine atom, or a iodine atom;
R is a protecting group; and
B is as defined above;
with at least one perfluoroalkanesulfonyl fluoride in the presence of at least one base to obtain a 2',3'-didehydro-2',3'-dideoxypurine nucleoside compound represented by formula (2) 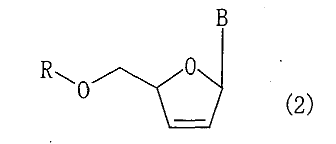 wherein:
R and B are as defined above; and
(b) subjecting said 2',3'-didehydro-2',3'-dideoxypurine nucleoside compound of formula (2) to de-protection of R and, if necessary, to at least one of protection, de-protection and modification for a group at the 2-position and/or the 6-position of the purine base.

12. A process for the production of a 2',3'-didehydro-2',3'-dideoxypurine nucleoside compound represented by formula (6') 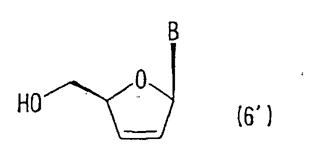 wherein:
B is a purine base;
wherein said process comprises:
(a) treating a 3'-deoxy-3'-halopurine nucleoside compound represented by formula (1') 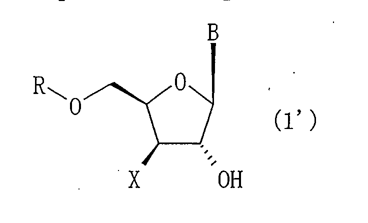 wherein:
X is a chlorine atom, a bromine atom, or a iodine atom;
R is a protecting group; and
B is as defined above;
with at least one perfluoroalkanesulfonyl fluoride in the presence of at least one base to obtain a 2',3'-didehydro-2',3'-dideoxypurine nucleoside compound represented by formula (2'): 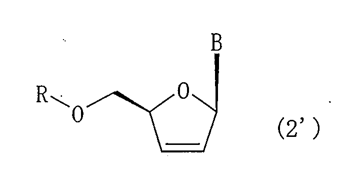 wherein:
R and B are as defined above; and
(b) subjecting .said 2',3'-didehydro-2',3'-dideoxypurine nucleoside compound of formula (2') to de-protection of R and, if necessary, to at least one of protection, deprotection and modification for a group at the 2-position and/or the 6-position of the purine base.

13. A process for the production of a 2',3'-dideoxypurine nucleoside compound represented by formula (7) 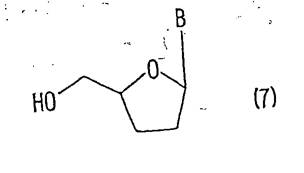 wherein
B is a purine base;
wherein said process comprises:
(a) treating a 3'-deoxy-3'-halopurine nucleoside compound represented by formula (1): 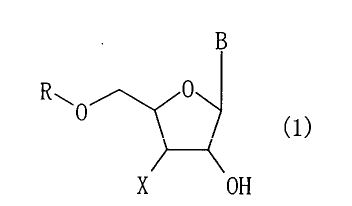 wherein:
X is a chlorine atom, a bromine atom, or a iodine atom;
R is a protecting group; and
B is as deined above;
with at least one perfluoroalkanesulfonyl fluoride in the presence of at least one base to obtain a 2',3'-didehydro-2',3'-dideoxypurine nucleoside compound represented by formula (2) 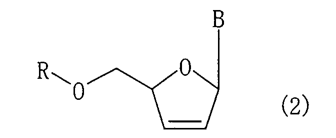 wherein:
R and B are as defined above;
(b) subjecting said 2',3'-didehydro-2',3'-dideoxypurine nucleoside compound of formula (2) to catalytic hydrogenation to obtain said 2',3'-dideoxypurine nucleoside compound of formula (3) 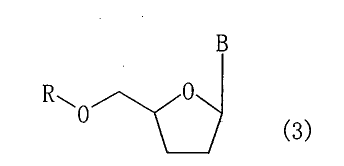 wherein:
R and B are as defined above; and
(c) subjecting said 2',3'-dideoxypurine nucleoside compound of formula (3) to de-protection of R and, if necessary, to at least one of protection, de-protection and modification for a group at the 2-position and/or the 6-position of the purine base.

14. A process for the production of a 2',3'-dideoxypurine nucleoside compound represented by formula (7') 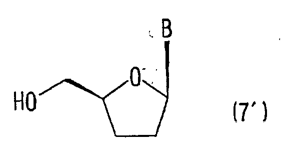 wherein:
B is a purine base;
wherein said process comprises:
(a) treating a 3'-deoxy-3'-halopurine nucleoside compound represented by formula (1'): 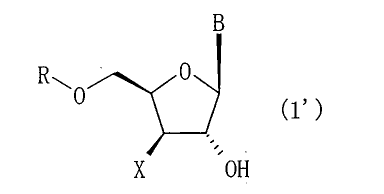 wherein:
X is a chlorine atom, a bromine atom or a iodine atom;
R is a protecting group; and
B is as defined above
with at least one perfluoroalkanesulfonyl fluoride in the presence of at least one base to obtain a 2',3'-didehydro-2',3'-dideoxypurine nucleoside compound represented by formula (2'): 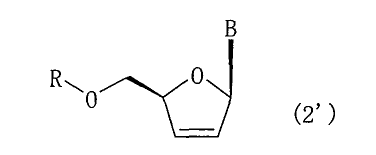 wherein:
R and B are as defined above;
(b) subjecting said 2',3'-didehydro-2',3'-dideoxypurine nucleoside compound of formula (2) to catalytic hydrogenation to obtain said 2',3'-dideoxypurine nucleoside compound of formula (3'): 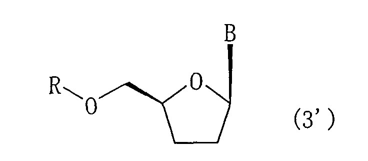 wherein
R and B are as defined above; and
(c) subjecting said 2',3'-dideoxypurine nucleoside compound of formula (3) to de-protection of R and, if necessary, to at least one of protection, de-protection and modification for a group at the 2-position and/or the 6-position of the purine base.
